# NOUVEAU FASCICULE DE BREVET EUROPEEN

(11) **EP 0 498 740 B2**
(45) Date de publication et mention de la décision concernant l'opposition: **15.10.1997**
(45) Mention de la délivrance du brevet: 07.12.1994
(21) Numéro de dépôt: 92420010.8
(22) Date de dépôt: 10.01.1992
(51) Int. Cl.: A61M 1/36

(54) **Dispositif et procédé de mise à niveau d'un liquide dans une chambre d'un circuit extracorporel de sang**
Vorrichtung und Verfahren zum Nivellieren einer Flüssigkeit in einer Kammer eines extrakorporalen Blutkreislaufs
Extracorporal blood circuit chamber liquid levelling device and process

(30) Priorité: 06.02.1991 FR 9101564
(43) Date de publication de la demande: 12.08.1992
(73) Titulaire: HOSPAL INDUSTRIE, F-69883 Meyzieu Cédex (FR)
(72) Inventeur: Riquier, Jean-Claude, F-69140 Rilleux (FR)

(56) Documents cités:
- EP-A- 0 044 694
- EP-A- 0 104 895
- EP-A- 0 222 709
- DE-A- 3 418 434
- DE-A- 3 923 079
- FR-A- 2 242 994
- GB-A- 2 141 936
- US-A- 3 756 234
- US-A- 3 946 731
- US-A- 3 964 479
- IBM Technical Disclosure Bulletin, Vol.19, No.3, August 1976, pages 765 - 768

## Description

La présente invention concerne le domaine technique de la circulation extracoporelle du sang en vue de son traitement par un dispositif approprié. Plus particulièrement, la présente invention concerne un dispositif comprenant un circuit extracorporel de sang comprenant au moins une chambre contenant du liquide dans sa partie inférieure et de l'air dans sa partie supérieure, cette chambre étant reliée à une pompe apte à y faire varier la quantité d'air.

On connait dans l'art antérieur de tels dispositifs utilisés notamment pour effectuer le traitement du sang par dialyse. Ainsi, le brevet US 4 490 135 décrit un circuit extracorporel de sang comportant une portion artérielle destinée à conduire le sang d'un patient à un hémodialyseur et une portion veineuse destinée à assurer le retour du sang traité au patient. "La portion veineuse comprend une poche souple pour servir de réservoir tampon pour le sang du patient, le réservoir étant alternativement rempli et vidé au cours du fonctionnement de ce circuit" Chacune des portions artérielle et veineuse comporte une chambre dont la partie supérieure est reliée à une pompe permettant grâce à l'addition ou au retrait d'air d'ajuster le niveau de liquide dans la chambre. Cependant, la mise en oeuvre de la pompe est commandée manuellement en fonction du niveau de liquide repéré visuellement par l'opérateur. Or, cette surveillance visuelle représente une contrainte importante pour l'utilisateur qui ne peut pas l'exercer de façon permanente. En effet, dans un centre de traitement tel qu'un centre de dialyse, une seule infirmière a la charge de surveillance de plusieurs postes ; elle doit donc surveiller à la fois la bonne marche de plusieurs machines ainsi que l'état de santé des patients. Cependant, le maintien du niveau de liquide dans les chambres est essentiel pour un traitement efficace et sûr. Il faut d'une part éviter les débordements de sang et d'autre part, maintenir un niveau minimum de liquide ; en effet, la baisse trop importante du niveau de liquide dans la chambre située sur la portion artérielle pourrait conduire à l'introduction d'air dans l'hémodialyseur, ce qui réduirait fortement son efficacité, alors que la baisse du niveau de liquide dans la chambre veineuse pourrait conduire à l'introduction d'air dans le réseau sanguin du patient, ce qui peut avoir des conséquences graves. Il est donc important de pouvoir assurer de façon permanente une surveillance et une correction du niveau de liquide dans les chambres sans contrainte pour l'utilisateur et avec une très grande sécurité.

Pour atteindre ce but, on prévoit, conformément à l'invention un dispositif tel que défini dans la revendication 1.

D'autres caractéristiques de l'invention sont définies dans les sous-revendications, qui présentent les avantages suivants:

La détection d'un niveau bas et d'un niveau haut dans chaque chambre permet, de contrôler la variation de volume existant dans la chambre entre la phase où le sang est aspiré du patient (phase artérielle) et la phase où le sang est restitué au patient (phase veineuse).

Aussi, le volume de liquide disponible pour le traitement, c'est-à-dire la différence entre le volume de liquide présent dans le circuit à la fin de la phase artérielle et le volume de liquide présent dans le circuit à la fin de la phase veineuse, est parfaitement contrôlé, ce qui permet d'optimiser l'efficacité du traitement.

Aussi, on peut choisir on peut choisir le niveau souhaité de liquide à l'intérieur de la chambre, ainsi que l'écart admissible entre ce niveau souhaité et le niveau véritablement atteint par le liquide. L'utilisation d'une plage de consigne évite, dans le cas où le niveau réel est très proche du niveau souhaité, d'induire une oscillation du niveau de liquide entre un niveau légèrement supérieur au niveau souhaité et un niveau légèrement inférieur au niveau souhaité, en mettant la pompe en oeuvre alternativement dans un sens puis dans l'autre.

L'invention a également pour objet un procédé de mise à niveau de liquide tel que défini dans la revendication 11

La présente invention sera mieux comprise à la lecture de la description qui va suivre, en référence aux dessins annexés qui illustrent, à titre d'exemple, différents modes de réalisation de la présente invention.

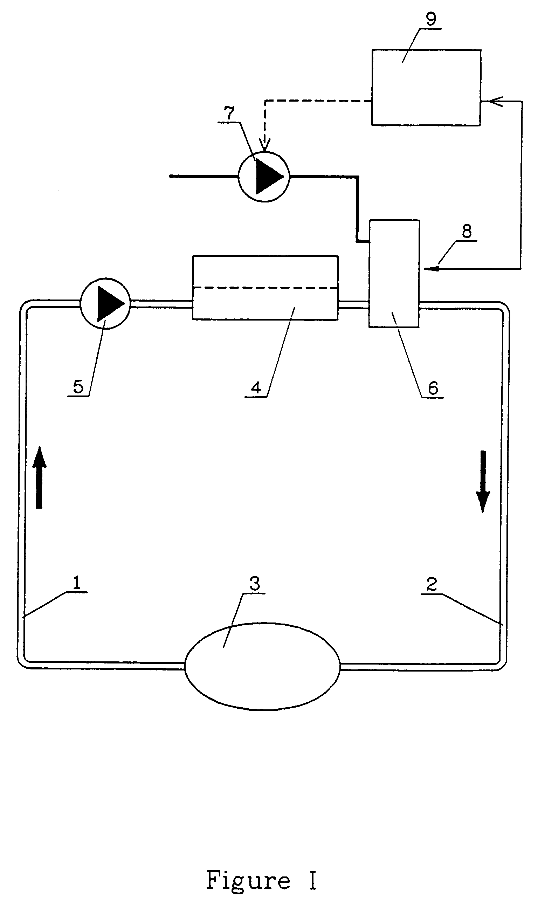

La figure 1 est une illustration qui aide à la compréhension de l'invention.

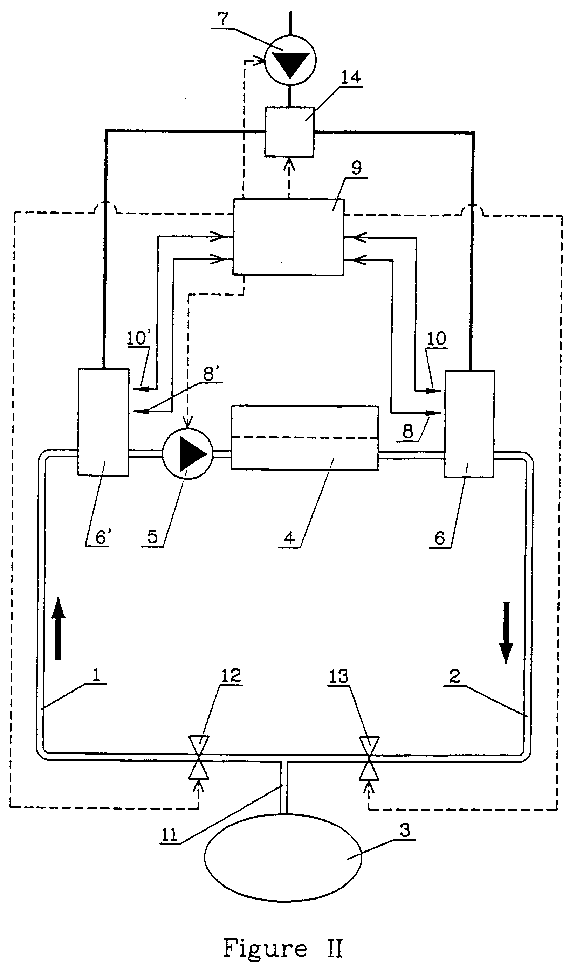

La figure 2 illustre un mode de réalisation de l'invention, en relation avec un circuit extracoporel de sang dit d'aiguille unique.

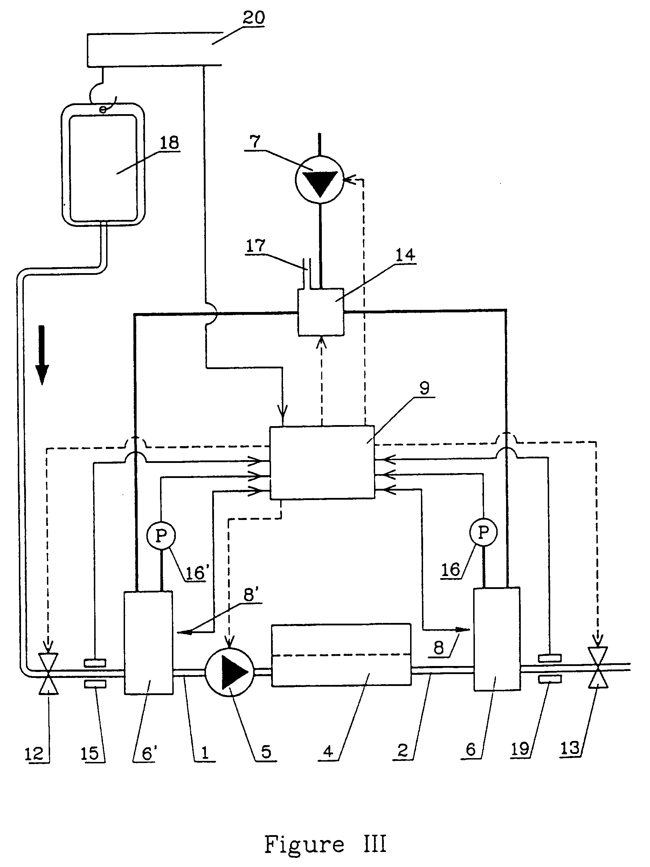

La figure 3 illustre l'objet de l'invention utilisé dans le cadre du remplissage automatique d'un circuit extracorporel de sang.

Le circuit extracorporel de sang représenté sur la figure I comporte de façon classique, une portion artérielle 1 et une portion veineuse 2. La portion artérielle 1 conduit le sang provenant d'un patient 3 vers un dispositif de traitement 4 constitué par exemple par un hémodialyseur, un oxygénateur, un dispositif de plasmaphérèse ou tout autre type de dispositif de traitement connu en soi. Aux fins de la présente description, on choisit un hémodialyseur apte à traiter le sang par dialyse et/ou par ultrafiltration. Une pompe 5 assure la mise en circulation du sang dans l'ensemble du circuit.

De façon classique, la portion veineuse 2 comporte une chambre de dégazage 6 contenant du liquide dans sa partie inférieure et de l'air dans sa partie supérieure. Cette chambre est reliée à une pompe 7 dont la mise en oeuvre permet de modifier la quantité d'air à l'intérieur de la chambre. Ainsi, grâce à l'action de la pompe 7 qui permet d'ajouter ou de retirer de l'air dans la chambre, il est possible de faire varier le niveau de liquide à l'intérieur de la chambre.

La chambre de dégazage 6 est équipée d'un capteur de niveau 8 capable de produire un signal correspondant au niveau de liquide à l'intérieur de la chambre. Il s'agit par exemple d'un capteur à fibres optiques émettant un signal proportionnel au niveau de liquide. Le signal produit par le capteur 8 est transmis à une unité de contrôle 9 qui comporte des moyens pour comparer le signal produit avec un signal de consigne correspondant à un niveau de consigne du liquide ; l'unité 9 comporte également des moyens pour commander la pompe 7 quand le signal produit ne correspond pas au signal de consigne.

Ainsi, dans le cas où le signal produit par le capteur 8 correspond à un état où le niveau de liquide dans la chambre 6 est inférieur au niveau de consigne, la pompe 7 est commandée pour retirer de l'air de la chambre 6 jusqu'à ce que le liquide atteigne le niveau de consigne.

Par contre, dans le cas où le signal produit par le détecteur 8 correspond à un état où le niveau de liquide dans la chambre est supérieur au niveau de consigne, la pompe 7 est commandée pour ajouter de l'air dans la chambre jusqu'à ce que le niveau de liquide redescende au niveau de consigne.

Afin d'éviter au niveau de liquide dans la chambre d'osciller de part et d'autre du niveau de consigne par mise en oeuvre de la pompe 7 successivement dans un sens puis dans l'autre, il est possible de comparer le signal produit par le capteur 8 non pas avec une valeur de consigne précise mais avec un ensemble de valeurs constituant une plage de consigne, les bornes de cette plage constituant les limites admissibles au-delà desquelles la pompe 7 est mise en oeuvre.

Ainsi, la pompe 7 n'est commandée que dans le cas où le niveau de liquide s'écarte du niveau souhaité de façon trop importante.

Dans une variante, le capteur de niveau utilisé permet simplement de détecter la présence ou non de liquide à son niveau grâce par exemple à une détection du type optique ; il est alors avantageux de munir la chambre de dégazage 6 d'un second détecteur de niveau. Dans ce cas, l'unité 9 commande la pompe 7 de façon à maintenir le niveau de liquide dans la chambre entre une limite inférieure marquée par un premier détecteur appelé détecteur de niveau bas et une limite supérieure marquée par un second détecteur appelé détecteur de niveau haut. En effet, quand le niveau de liquide dans la chambre atteint le détecteur de niveau bas, le signal produit par le détecteur est transmis à l'unité 9 qui commande le fonctionnement de la pompe 7 pour retirer de l'air de la chambre ; à l'inverse, lorsque le niveau de liquide dans la chambre atteint le détecteur de niveau haut, celui-ci produit un signal transmis à l'unité 9 qui commande le fonctionnement de la pompe 7 pour ajouter de l'air dans la chambre. Ainsi, le niveau de liquide est constamment maintenu entre deux limites choisies par le positionnement des deux détecteurs de niveau qui constituent les bornes de la plage de consigne du niveau souhaité de liquide.

Selon le mode de réalisation représenté sur la figure II, le dispositif objet de la présente invention est utilisé en relation avec un circuit extracorporel de sang dit d'aiguille unique, c'est-à-dire que le sang est prélevé du patient 3 et restitué au patient par un accès unique 11 à l'intérieur duquel la circulation s'effectue alternativement dans un sens puis dans l'autre.

Dans le cas représenté ici, un clamp 12 ou tout autre dispositif d'obturation permet de fermer l'entrée de la portion artérielle 1 , de même un clamp 13 permet de fermer la sortie te la portion veineuse 2.

La portion artérielle 1 comporte une chambre de dégazage 6' dont la capacité est choisie gour que la chambre puisse être utilisée comme réservoir-tampon ; en aval de la chambre 6' une pompe 5 assure la circulation du sang.

La portion veineuse 2 comporte également une chambre de dégazage 6 dont la capacité est choisie gour que la chambre puisse être utilisée comme réservoir-tampon.

Selon l'invention, chacune des chambres de dégazage 6 et 6' est munie d'un détecteur de niveau bas 8 et 8' et d'un détecteur de niveau haut 10 et 10'. Les détecteurs 8, 8', 10 et 10' sont reliés à une unité 9 qui commande une pompe 7 apte à faire varier la quantité d'air à l'intérieur de chacune des chambres. De façon avantageuse on utilise une seule pompe 7 qui peut être mise en relation sélectivement, soit avec la chambre artérielle 6', soit avec la chambre veineuse 6 grâce par exemple à un distributeur 14 commandé par l'unité 9. En outre, l'unité 9 commande l'alternance d'ouvertures et de fermetures des clamps artériel et veineux 12 et 13, au rythme de l'alternance des phases artérielles et veineuses.

Le fonctionnement du circuit extracorporel représenté ici est le suivant. Les phases d'aspiration du sang ou phases artérielles et les phases de restitution ou phases veineuses se succèdent alternativement. Ainsi, en phase artérielle, le champ 12 est ouvert alors que le clamp 13 est fermé. Le sang est aspiré du patient 3 ; le niveau de liquide monte à l'intérieur te chacune des chambres de dégazage 6 et 6'. Par contre, en phase veineuse, le clamp 12 est fermé et le clamp 13 ouvert. Le fonctionnement de la pompe 5 est maintenu et le sang est restitué au patient. Le niveau de liquide baisse alors à l'intérieur des chambres 6 et 6'.

Afin d'obtenir de l'hémodialyseur 4 une efficacité optimale durant le temps de traitement, il est important de s'assurer que le niveau de liquide atteint le niveau haut dans chaque chambre à la fin de chaque phase artérielle et le niveau bas dans chaque chambre à la fin de chaque phase veineuse.

Le dispositif objet de la présente invention est particulièrement bien adapté pour effectuer cette commande.

Ainsi, par exemple, si on choisit de déclencher la phase artérielle, c'est-à-dire la fermeture du clamp 13 et l'ouverture du clamp 12 lorsque le liquide atteint le détecteur de niveau bas 8' dans la chambre artérielle 6', et le déclenchement de la phase veineuse, c'est-à-dire l'ouverture du clamp 13 et la fermeture du clamp 12 lorsque le liquide atteint le détecteur de niveau haut 10 dans la chambre veineuse 6, la maîtrise des volumes à l'intérieur des chambres est effectuée de la façon suivante.

Lorsque, à la fin de la phase veineuse, le liquide atteint le détecteur de niveau bas 8' dans la chambre artérielle, l'unité de commande 9 déclenche le passage à la phase artérielle par fermeture du clamp 13 et ouverture du clamp 12 ; l'unité 9 démarre alors une mesure du temps t qui va s'écouler jusqu'à ce que le liquide dans le chambre veineuse atteigne le détecteur de niveau haut 10, ce qui va alors déclencher le passage à la phase veineuse. Ce temps t mesuré est ensuite comparé à une valeur théorique th. Cette valeur th est déterminée par l'unité de contrôle 9 en fonction du débit de la pompe 5, du volume de la chambre entre le niveau du détecteur 8 et le niveau du détecteur 10, et des conditions de pression à l'intérieur de la chambre ; th représente alors le temps que met le liquide à s'élever du niveau bas représenté par le détecteur 8 au niveau haut représenté par le détecteur 10 dans les conditions de fonctionnement choisies.

Lorsque la valeur mesurée t s'écarte d'une plage de consigne entourant la valeur théorique th, l'unité 9 commande la mise en oeuvre de la pompe 7 afin de modifier la quantité d'air existant à l'intérieur de l'une des chambres ou des deux. Il est possible de vérifier l'efficacité de la correction et de l'ajuster si nécessaire, en effectuant une autre mesure au cycle suivant. Il est également possible de déclencher la mesure du temps sur la chambre artérielle à partir du moment où le liquide atteint le détecteur de niveau haut 10 dans la chambre veineuse.

Au lieu d'utiliser un détecteur de niveau haut et un détecteur de niveau bas dans chaque chambre, on peut également utiliser un seul capteur de niveau, par exemple à fibres optiques, permettant de connaître, à chaque instant, le niveau du liquide dans la chambre. Dans ce cas, il n'est plus nécessaire d'effectuer de mesure du temps ; en effet, il suffit alors, de comparer les signaux provenant des capteurs de niveau de chaque chambre à la fin de la phase artérielle et/ou de la phase veineuse, avec les signaux de consigne correspondants.

Dans le cas où les signaux produits se situent en dehors des plages de consigne prédéterminées, l'unité de contrôle 9 commande la mise en oeuvre de la pompe 7 et du distributeur 14 afin de faire varier la quantité d'air dans l'une et/ou l'autre des chambres 6 et 6'.

La détermination du sens d'action de la pompe 7 peut être effectuée de la manière suivante ; lorsque, à la fin de la phase artérielle, le liquide n'atteint pas le niveau haut souhaité dans une chambre, il faut commander la pompe 7 de manière à retirer de l'air de cette chambre. Par contre, lorsque à la fin de la phase veineuse, le liquide n'atteint pas le niveau bas souhaité dans une chambre, la pompe 7 doit être commandée de manière à ajouter de l'air dans cette chambre.

L'unité de contrôle 9 peut être consituée par un microprocesseur.

Selon le mode de réalisation représenté sur la figure III, le dispositif objet de l'invention est particulièrement bien adapté pour le rinçage et le remplissage automatiques d'un circuit extracorporel de sang. Sur cette figure, les éléments communs aux figures précédemment décrites portent les mêmes références numériques. La portion artérielle 1 comporte un détecteur de liquide 15 situé en amont et à proximité de la chambre 6'. La portion veineuse 2 comporte un détecteur de sang 19 situé en aval de la chambre 6 et en amont du clamp 13. Chaque chambre de dégazage comporte avantageusement un capteur de pression 16 et 16' transmettant à l'unité de contrôle 9 les valeurs de pression mesurées.

Le distributeur 14 permet de mettre la pompe 7 en communication avec la chambre artérielle 6' ou la chambre veineuse 6. Il permet en outre, grâce à une ouverture 17, de relier l'une ou l'autre des chambres 6 et 6' avec l'atmosphére.

Afin d'assurer le rinçage et le remplissage du circuit extracorporel représenté ici, la portion artérielle 1 est reliée à une poche de liquide physiologique stérile 18 qui peut être accrochée à une balance 20 dont les informations sont transmises à l'unité de contrôle 9 qui peut ainsi contrôler la quantité de liquide de rinçage utilisé. Bien que cela ne soit pas représenté, la portion veineuse 2 est reliée à une poche de recueil ou à des moyens d'évacuation du liquide de rinçage usagé.

Le liquide est mis en circulation dans la portion artérielle grâce au fonctionnement des pompes 5 et 7 et éventuellement de la mise en charge de la poche 18. A cette fin, la position du distributeur 14 est telle que la pompe 7 est en communication avec la chambre artérielle 6' ; les clamps 12 et 13 sont ouverts. Lorsque le liquide physiologique atteint le détecteur 15, celui-ci avertit l'unité de contrôle 9 qui commande l'arrêt de la pompe 5. La pompe 7 continue à fonctionner jusqu'au moment où le niveau de liquide dans la chambre artérielle 6' atteint le capteur 8' qui produit alors un signal transmis à l'unité 9 qui commande à nouveau la mise en oeuvre de la pompe 5 et agit sur le distributeur 14 afin de mettre en communication la pompe 7 et la chambre veineuse 6. Le liquide physiologique s'écoule alors dans l'hémodialyseur 4 et la chambre veineuse 6 jusqu'à atteindre le niveau du capteur 8 qui engendre un signal transmis à l'unité 9. L'unité 9 commande alors l'arrêt de la pompe 7. La pompe 5 continue à assurer la circulation du liquide de rinçage à l'intérieur du circuit jusqu'à ce qu'une quantité suffisante de liquide ait circulé.

Selon un mode particulier du procédé de rinçage et de remplissage du circuit extracorporel de sang avec le dispositif objet de l'invention, lorsque le liquide atteint le détecteur de niveau 8' dans la chambre artérielle 6', l'unité de contrôle 9 commande la fermeture du champ 13 et agit sur le distributeur 14 afin de mettre la chambre 6 en communication avec la pompe 7 dont l'action est commandée pour retirer de l'air de la chambre. L'action de la pompe 7 est maintenue jusqu'à ce que la dépression créée à l'intérieur de la chambre atteigne une valeur prédéterminée qui correspond à des conditions de détente de l'air à l'intérieur de l'hémodialyseur favorables à un bon dégazage de celui-ci lors de son remplissage. Lorsque la pression mesurée par le capteur 16 atteint la valeur prédéterminée, l'unité de contrôle 9 commande à nouveau le fonctionnement de la pompe 5. Le liquide de rinçage remplit alors le compartiment sang de l'hémodialyseur 4 puis atteint le détecteur de niveau 8. A ce moment, l'unité de contrôle 9 commande l'arrêt de la pompe 7, l'ouverture du champ 13 et agit sur le distributeur 14 de manière à mettre, un très court instant, la chambre 6 en relation avec l'atmosphére.

Le rinçage du circuit se poursuit ensuite grâce au fonctionnement de la pompe 5.

Il est alors possible d'ajuster la pression existant à l'intérieur de chaque chambre 6 et 6'. En effet, les informations provenant des capteurs de pression 16 et 16' sont transmises à l'unité de contrôle 9 qui peut les comparer avec des valeurs de consigne ou des plages de consigne et agir sur la pompe 7 et le distributeur 14 afin de faire varier la quantité d'air à l'intérieur des chambres pour obtenir la pression souhaitée.

Lorsque le circuit est suffisamment rincé, ce qui peut être directement déterminé par l'unité de contrôle 9 grâce aux informations provenant de la balance 20, les champs 12 et 13 sont fermés et la pompe 5 arrêtée ; la portion artérielle 1 du circuit extracorporel est alors reliée à une source de sang à traiter, par exemple à un patient.

La pompe 5 est ensuite remise en route et les champs 12 et 13 sont ouverts ; le sang mis en circulation dans le circuit pousse alors le liquide de rinçage jusqu'à ce que la présence de sang soit détectée par un détecteur 19 présent sur la portion veineuse 2 en aval de la chambre 6. Les clamps 12 et 13 sont alors fermés et la portion veineuse 2 reliée au patient.

Après ouverture des clamps 12 et 13, le traitement du sang par hémodialyse peut alors véritablement commencer, que le circuit extracorporel du sang soit utilisé en mode de double aiguille ou de simple aiguille. Dans ce dernier cas, il est possible d'utiliser à nouveau la pompe 7 pour corriger la pression à l'intérieur des chambre 6 et 6', si cela s'avère nécessaire en fonction des informations fournies par les capteurs 16 et 16'.

## Revendications

1. Dispositif de traitement du sang par circulation extracorporelle dans un circuit comprenant :
- une canalisation artérielle (1), sur laquelle est disposée une chambre artérielle (6'), pour relier un patient à un appareil de traitement (4) ;
- une canalisation veineuse (2), sur laquelle est disposée une chambre veineuse (6), pour relier l'appareil de traitement (4) au patient ;
- au moins une pompe (5) pour faire circuler un liquide dans le circuit;
- des moyens (7, 14) pour faire varier la quantité d'air dans chaque chambre (6, 6') ;
- au moins un moyen de détection de niveau (8, 10 ; 8', 10') par chambre (6, 6'), pour produire un signal correspondant à un niveau bas déterminé de liquide et un niveau haut déterminé de liquide dans chaque chambre (6, 6') ; et
- des moyens de commande (9) pour commander, en réponse au signal produit par les moyens de détection de niveau (8, 10; 8', 10'), les moyens (7, 14) pour faire varier la quantité d'air dans chaque chambre (6, 6') de façon que, en fonctionnement, le niveau du liquide mis en circulation varie entre le niveau bas et le niveau haut dans chaque chambre (6, 6'), et inversement.

2. Dispositif selon la revendication 1, caractérisé en ce que les moyens de commande (9) sont prévus pour commander les moyens (7, 14) pour faire varier la quantité d'air dans chaque chambre (6, 6') de façon que le liquide mis en circulation atteigne simultanément le niveau bas dans les deux chambres (6, 6') et atteigne simultanément le niveau haut dans les deux chambres (6, 6').

3. Dispositif selon une des revendications 1 et 2, caractérisé en ce que les moyens de commande (9) sont prévus pour commander un ajout d'air, par les moyens (7, 14) pour faire varier la quantité d'air, dans la chambre (6, 6') où le niveau de liquide n'a pas atteint le niveau bas à la fin d'une phase de restitution du sang au patient.

4. Dispositif selon une des revendications 1 à 3, caractérisé en ce que les moyens de commande (9) sont prévus pour commander un retrait d'air, par les moyens (7, 14) pour faire varier la quantité d'air, hors de la chambre (6, 6') où le niveau de liquide n'a pas atteint le niveau haut à la fin d'une phase d'aspiration de sang hors du patient.

5. Dispositif selon une des revendications 1 à 4, caractérisé en ce que les moyens pour faire varier la quantité d'air dans chaque chambre (6, 6') comprennent une pompe (7) et des moyens de distribution (14) pour mettre la pompe (7) sélectivement en communication avec la chambre artérielle (6') et avec la chambre veineuse (6).

6. Dispositif selon une des revendications 1 à 5, caractérisé en ce que les moyens de commande (9) comprennent :
- des moyens pour fixer, pour un niveau bas déterminé dans chaque chambre (6, 6'), une valeur de consigne du signal produit par les moyens de détection de niveau (8, 10 ; 8', 10'), et pour fixer, pour un niveau haut déterminé dans chaque chambre (6, 6'), une valeur de consigne du signal produit par les moyens de détection de niveau (8, 10 ; 8', 10') ;
- des moyens pour définir une plage de consigne entourant chaque valeur de consigne ;
- des moyens pour vérifier si le signal émis par les moyens de détection de niveau (8, 10 ; 8', 10') pour chaque chambre (6, 6') se situe à l'intérieur d'une plage de consigne ;
- des moyens pour déterminer s'il faut ajouter ou retirer de l'air dans chaque chambre (6, 6') lorsque le signal émis par les moyens de détection de niveau (8, 10 ; 8', 10') se situe à l'extérieur d'une plage de consigne.

7. Dispositif selon une des revendications 1 à 6, caractérisé en ce que les moyens pour détecter dans chaque chambre (6, 6') un niveau bas de liquide et un niveau haut de liquide comprennent un détecteur de niveau haut (10, 10') et un détecteur de niveau bas (8, 8') associés à chaque chambre (6, 6').

8. Dispositif selon une des revendications 1 à 6, caractérisé en ce que les moyens pour détecter dans chaque chambre (6, 6') un niveau bas de liquide et un niveau haut de liquide comprennent deux détecteurs de niveau (8, 10 ; 8', 10') associé à chaque chambre (6, 6') pour détecter respectivement la présence ou l'absence de liquide à un niveau bas et à un niveau haut prédéterminés.

9. Dispositif selon une des revendications 1 à 8, caractérisé en ce les moyens de commande sont constitués par un microprocesseur (9).

10. Dispositif selon une des revendications 1 à 9, caractérisé en ce qu'il comporte
- des moyens (16, 16') de mesure de pression pour mesurer la pression dans chaque chambre (6, 6') ;
- des moyens pour fixer une valeur de consigne de pression pour chaque chambre (6, 6') ;
- des moyens pour déterminer une plage de consigne entourant chaque valeur de consigne ;
- des moyens pour vérifier si la pression mesurée dans chaque chambre (6, 6') se situe à l'intérieur de la plage de consigne correspondante ;
- des moyens pour déterminer le sens de la variation de la quantité d'air à imposer dans chaque chambre (6, 6') lorsque la pression mesurée se situe à l'extérieur de la plage de consigne correspondante.

11. Procédé de mise à niveau d'un liquide présent dans une chambre (6') située sur un circuit extracorporel de sang en amont d'un dispositif de traitement du sang (4) ainsi que d'un liquide présent dans une chambre (6) située sur le circuit extracorporel de sang en aval du dispositif de traitement du sang (4), le procédé étant du type à ajouter ou à retirer de l'air dans chaque chambre (6, 6') au moyen d'une pompe (7),
caractérisé en ce qu'il comporte les étapes suivantes :
- déterminer, dans chaque chambre (6, 6'), l'atteinte par le liquide d'un niveau inférieur et d'un niveau supérieur au moyen d'un signal provenant d'un capteur de niveau (8, 8' ; 10, 10') ;
- comparer, pour chaque chambre (6, 6'), le signal provenant du capteur de niveau (8, 8' ; 10, 10') avec un signal de consigne ;
- déterminer, pour chaque chambre, en fonction du résultat de la comparaison, le sens d'action de la pompe (7), c'est-à-dire s'il faut ajouter de l'air dans la chambre (6, 6') ou en retirer ;
- commander la pompe dans le sens déterminé de façon que, en fonctionnement, le niveau du liquide varie entre le niveau inférieur et le niveau supérieur dans chaque chambre (6, 6'), et inversement.

12. Procédé selon la revendication 11, caractérisé en ce qu'il consiste à commander la pompe (7) de façon que le liquide atteigne simultanément le niveau inférieur dans les deux chambres (6, 6') et le niveau supérieur dans les deux chambres (6, 6').

13. Procédé selon une des revendications 11 ou 12, caractérisé en ce qu'il consiste en outre à :
- déterminer une plage de consigne entourant le signal de consigne ;
- comparer le signal provenant du capteur de niveau (8, 8', 10, 10') avec la plage de consigne ;
- commander la mise en oeuvre de la pompe (7) si le signal provenant du capteur de niveau se situe à l'extérieur de la plage de consigne.

## Claims

1. Apparatus for the treatment of blood by extracorporeal circulation in a circuit comprising:
- an arterial line (1), on which an arterial chamber (6') is arranged, for connecting a patient to a treatment device (4);
- a venous line (2), on which a venous chamber (6) is arranged, for connecting the treatment device (4) to the patient;
- at least one pump (5) to circulate a liquid in the circuit;
- means (7, 14) for varying the quantity of air in each chamber (6, 6');
- at least a level detection means (8, 10; 8', 10') for each chamber (6, 6') for generating a signal corresponding to a determined low level of liquid and a determined high level of liquid in each chamber (6, 6'); and
- control means (9) for controlling, in response to the signal generated by the level detection means (8, 10; 8', 10'), the means (7, 14) for varying the quantity of air in each chamber (6, 6') so that, in operation, the level of the liquid being circulated varies between the low level and the high level in each chamber (6, 6') and conversely.

2. Apparatus according to Claim 1, characterized in that the control means (9) is designed to control the means (7, 14) for varying the quantity of air in each chamber (6, 6') so that the liquid being circulated reaches simultaneously the low level in both chambers (6, 6') and simultaneously reaches the high level in both chambers (6, 6').

3. Apparatus according to one of Claims 1 and 2, characterized in that the control means (9) is designed to cause the addition of air, through the means (7, 14) for varying the quantity of air, in the chamber (6, 6') where the level of liquid has not reached the low level in the end of a phase of returning blood to the patient.

4. Apparatus according to one of Claims 1 to 3, characterized in that the control means (9) is designed to cause the withdrawal of air, through the means (7, 14) for varying the quantity of air, from the chamber (6, 6') where the level of liquid has not reached the high level in the end of a phase of drawing blood from the patient.

5. Apparatus according to one of Claims 1 to 4, characterized in that the means for varying the quantity of air in each chamber (6, 6') comprises a pump (7) and distribution means (14) for selectively connecting the pump (7) to the arterial chamber (6') and to the venous chamber (6).

6. Apparatus according to one of Claims 1 to 5, characterized in that the control means (9) comprises :
- means for fixing, for a determined low level in each chamber (6, 6'), a reference value for the signal generated by the level detection means (8, 10; 8', 10'), and for fixing, for a determined high level in each chamber (6, 6'), a reference value for the signal generated by the level detection means (8, 10; 8', 10');
- means for defining a range of reference values surrounding each reference value;
- means for checking whether the signal emitted by the level detection means (8, 10; 8', 10') for each chamber (6, 6') is situated within a reference range;
- means for determining whether air must be added in or withdrawn from each chamber (6, 6') when the signal emitted by the level detection means (8, 10; 8', 10') is situated outside a reference range.

7. Apparatus according to one of Claims 1 to 6, characterized in that the means for detecting in each chamber (6, 6') a low level of liquid and a high level of liquid comprises a high level detector (10, 10') and a low level detector (8, 8') associated with each chamber (6, 6').

8. Apparatus according to one of Claims 1 to 6, characterized in that the means for detecting in each chamber (6, 6') a low level of liquid and a high level of liquid comprises two level detector (8, 10; 8', 10') associated with each chamber (6, 6') for respectively detecting the presence or the absence of liquid at predetermined low level and high level.

9. Apparatus according to one of Claims 1 to 8, characterized in that the control means is constituted by a microprocessor (9).

10. Apparatus according to one of Claims 1 to 9, characterized in that it comprises:
- pressure measuring means (16, 16') for measuring the pressure in each chamber (6, 6');
- means for fixing a pressure reference value for each chamber (6, 6');
- means for determining a reference range surrounding each reference value;
- means for checking whether the pressure measured in each chamber (6, 6') is situated within the corresponding reference range;
- means for determining the direction of the variation of the quantity of air which must be caused in each chamber (6, 6') when the measured pressure is situated outside the corresponding reference range.

11. Method for setting the level of a liquid in a chamber (6') arranged in an extracorporeal blood circuit upstream of a blood treatment device (4) as well as the level of a liquid in a chamber (6) arranged in the extracorporeal blood circuit downstream of the blood treatment device (4), the method being of the type consisting of adding air to, or withdrawing air from each chamber (6, 6') by means of a pump (7), the method comprising the steps of:
- determining in each chamber (6, 6') that the liquid has reached a lower level and an upper level by means of a signal from a level detector (8, 8'; 10, 10');
- comparing, for each chamber (6, 6'), the signal from the level detector (8, 8'; 10, 10') with a reference signal;
- determining for each chamber (6, 6'), in accordance with the result of the comparison, the direction of action of the pump (7), that is to say, whether air is to be added in or to be withdrawn from the chamber (6, 6');
- actuating the pump in the determined direction so that, in operation, the level of the liquid varies between the lower level and the upper level in each chamber (6, 6') and conversely.

12. Method according to Claim 11, characterized in that it consists in actuating the pump (7) so that the liquid reaches simultaneously the lower level in both chambers (6, 6') and simultaneously reaches the upper level in both chambers (6, 6').

13. Method according to one of Claims 11 or 12, characterized in that it further consists in :
- determining a reference range surrounding the reference signal,
- comparing the signal from the level detector (8, 8'; 10, 10') with the reference range,
- actuating the operation of the pump (7) if the signal from the level detector falls outside the reference range.

## Patentansprüche

1. Vorrichtung zur Blutbehandlung durch extrakorporale Zirkulation in einem Kreislauf, der umfaßt:
- eine arterielle Leitung (1) mit einer arteriellen Kammer (6') zum Anschluß eines Patienten an ein Behandlungsgerät (4);
- eine venöse Leitung (2) mit einer venösen Kammer (6) zum Anschluß des Behandlungsgeräts (4) an den Patienten;
- mindestens eine Pumpe (5) für die Zirkulation einer Flüssigkeit im Kreislauf;
- Mittel (7, 14) zur Veränderung der Luftmenge in jeder Kammer (6, 6');
- mindestens ein Niveauerfassungsmittel (8, 10; 8', 10') je Kammer (6, 6') zur Erzeugung eines Signals entsprechend einem bestimmten unteren Flüssigkeitsniveau und einem bestimmten oberen Flüssigkeitsniveau in jeder Kammer (6, 6'); und
- Steuermittel (9), mit denen als Reaktion auf das von den Niveauerfassungsmitteln (8, 10; 8', 10') erzeugte Signal die Mittel (7, 14) zur Änderung der Luftmenge in jeder Kammer (6, 6') so gesteuert werden, daß sich bei Betrieb das Niveau der zirkulierenden Flüssigkeit in jeder Kammer (6, 6') zwischen dem unteren Niveau und dem oberen Niveau und umgekehrt ändert.

2. Vorrichtung nach Anspruch 1, dadurch gekennzeichnet, daß die Steuermittel (9) dazu bestimmt sind, die Mittel (7, 14) zur Änderung der Luftmenge in jeder Kammer (6, 6') so zu steuern, daß die zirkulierende Flüssigkeit in beiden Kammern (6, 6') gleichzeitig das untere Niveau und in beiden Kammern (6, 6') gleichzeitig das obere Niveau erreicht.

3. Vorrichtung nach einem der Ansprüche 1 und 2, dadurch gekennzeichnet, daß die Steuermittel (9) dazu bestimmt sind, die Zufuhr von Luft durch die Mittel (7, 14) zur Änderung der Luftmenge in der Kammer (6, 6') zu steuern, in der das Flüssigkeitsniveau am Ende einer Rückleitungsphase von Patientenblut nicht das untere Niveau erreicht hat.

4. Vorrichtung nach einem der Ansprüche 1 bis 3, dadurch gekennzeichnet, daß die Steuermittel (9) dazu bestimmt sind, die Absaugung von Luft durch die Mittel (7, 14) zur Änderung der Luftmenge aus der Kammer (6, 6') zu steuern, in der das Flüssigkeitsniveau am Ende einer Ansaugphase von Patientenblut nicht das obere Niveau erreicht hat.

5. Vorrichtung nach einem der Ansprüche 1 bis 4, dadurch gekennzeichnet, daß die Mittel zur Änderung der Luftmenge in jeder Kammer (6, 6') eine Pumpe (7) und Absperrmittel (14) haben, um die Pumpe (7) wahlweise mit der arteriellen Kammer (6') und mit der venösen Kammer (6) zu verbinden.

6. Vorrichtung nach einem der Ansprüche 1 bis 5, dadurch gekennzeichnet, daß die Steuermittel (9) umfassen:
- Mittel, um für ein bestimmtes unteres Niveau in jeder Kammer (6, 6') einen Sollwert des von den Niveauerfassungsmitteln (8, 10; 8', 10') erzeugten Signals festzulegen und um für ein bestimmtes oberes Niveau in jeder Kammer (6, 6') einen Sollwert des von den Niveauerfassungsmitteln (8, 10; 8', 10') erzeugten Signals festzulegen;
- Mittel, um für jeden Sollwert einen Sollbereich festzulegen;
- Mittel, um zu überprüfen, ob das von den Niveauerfassungsmitteln (8, 10; 8', 10') für jede Kammer (6, 6') abgegebene Signal innerhalb eines Sollbereichs liegt;
- Mittel, um zu bestimmen, ob in jede Kammer (6, 6') Luft zugeführt oder abgesaugt werden muß, wenn das von den Niveauerfassungsmitteln (8, 10; 8', 10') abgegebene Signal außerhalb eines Sollbereichs liegt.

7. Vorrichtung nach einem der Ansprüche 1 bis 6, dadurch gekennzeichnet, daß die Mittel zur Erfassung eines unteren Flüssigkeitsniveaus und eines oberen Flüssigkeitsniveaus in jeder Kammer (6, 6') einen jeder Kammer (6, 6') zugeordneten Oberniveaudetektor (10, 10') und Niederniveaudetektor (8, 8') haben.

8. Vorrichtung nach einem der Ansprüche 1 bis 6, dadurch gekennzeichnet, daß die Mittel zur Erfassung eines unteren Flüssigkeitsniveaus und eines oberen Flüssigkeitsniveaus in jeder Kammer (6, 6') zwei jeder Kammer (6, 6') zugeordnete Niveaudetektoren (8, 10; 8', 10') haben, um an einem vorbestimmten unteren und oberen Niveau die Anwesenheit bzw. die Abwesenheit von Flüssigkeit zu erfassen.

9. Vorrichtung nach einem der Ansprüche 1 bis 8, dadurch gekennzeichnet, daß die Steuermittel aus einem Mikroprozessor (9) bestehen.

10. Vorrichtung nach einem der Ansprüche 1 bis 9, dadurch gekennzeichnet, daß sie umfaßt:
- Druckmeßmittel (16, 16') zur Messung des Drucks in jeder Kammer (6, 6');
- Mittel zur Festlegung eines Drucksollwerts für jede Kammer (6, 6');
- Mittel zur Bestimmung eines Sollbereichs für jeden Sollwert;
- Mittel, um zu überprüfen, ob der Meßdruck in jeder Kammer (6, 6') innerhalb des entsprechenden Sollbereichs liegt;
- Mittel zur Bestimmung der Änderungsrichtung der Luftmenge jeder Kammer (6, 6'), wenn der Meßdruck außerhalb des entsprechenden Sollbereichs liegt.

11. Verfahren zur Angleichung des Niveaus einer Flüssigkeit in einer vor einer Blutbehandlungsvorrichtung (4) gelegenen Kammer (6') eines extrakorporalen Blutkreislaufs sowie einer Flüssigkeit in einer hinter der Blutbehandlungsvorrichtung (4) gelegenen Kammer (6) des extrakorporalen Blutkreislaufs, wobei das Verfahren darin besteht, daß in jede Kammer (6, 6') mit Hilfe einer Pumpe (7) Luft zugeführt bzw. abgesaugt wird,
dadurch gekennzeichnet, daß es folgende Stufen umfaßt:
- mit Hilfe eines von einem Niveausensor (8, 8'; 10, 10') abgegebenen Signals Bestimmung, daß die Flüssigkeit in jeder Kammer (6, 6') ein unteres und ein oberes Niveau erreicht hat;
- je Kammer (6, 6') Vergleich des vom Niveausensor (8, 8'; 10, 10') abgegebenen Signals mit einem Sollsignal;
- entsprechend dem beim Vergleich erzielten Ergebnis für jede Kammer Bestimmung der Arbeitsrichtung der Pumpe (7), d.h. ob in die Kammer (6, 6') Luft zugeführt oder ob Luft abgesaugt werden muß;
- Steuerung der Pumpe in der festgelegten Richtung, so daß sich das Flüssigkeitsniveau bei Betrieb in jeder Kammer (6, 6') zwischen dem unteren Niveau und dem oberen Niveau und umgekehrt verändert.

12. Verfahren nach Anspruch 11, dadurch gekennzeichnet, daß es darin besteht, daß die Pumpe (7) so gesteuert wird, daß die Flüssigkeit gleichzeitig in den beiden Kammern (6, 6') das untere Niveau und gleichzeitig in den beiden Kammern (6, 6') das obere Niveau erreicht.

13. Verfahren nach einem der Ansprüche 11 oder 12, dadurch gekennzeichnet, daß es außerdem darin besteht, daß:
- um den Sollwert herum ein Sollbereich bestimmt wird;
- das vom Niveausensor (8, 8'; 10, 10') abgegebene Signal mit dem Sollbereich verglichen wird;
- die Einschaltung der Pumpe (7) betätigt wird, wenn das vom Niveausensor abgegebene Signal außerhalb des Sollbereichs liegt.
